# EUROPÄISCHE PATENTSCHRIFT

(11) **EP 3 025 760 B1**
(45) Veröffentlichungstag und Bekanntmachung des Hinweises auf die Patenterteilung: **02.03.2022**
(21) Anmeldenummer: 15195892.3
(22) Anmeldetag: 23.11.2015
(51) Int. Cl.: A61N 5/10

(54) **DESIGN FÜR EINE VEREINFACHTE ENTFERNUNG EINES BALLON APPLIKATORS**
DESIGN FOR SIMPLIFIED REMOVAL OF A BALLOON APPLICATOR
CONCEPTION D'UN ÉLOIGNEMENT SIMPLIFIÉ D'UN APPLICATEUR DE BALLON

(30) Priorität: 27.11.2014 DE 102014117430
(43) Veröffentlichungstag der Anmeldung: 01.06.2016
(73) Patentinhaber: Carl Zeiss Meditec AG, 07745 Jena (DE)
(72) Erfinder: RÖDER, Norman, 99441 Döbritschen (DE)
(74) Vertreter: PATERIS Patentanwälte PartmbB

(56) Entgegenhaltungen:
- WO-A1-01/12255
- WO-A1-97/37714
- US-A1- 2005 080 340
- US-A1- 2011 071 506

## Beschreibung

Die Erfindung betrifft einen Ballon-Applikator für eine Bestrahlung eines Hohlraums innerhalb eines lebenden Gewebes, bei dem der Ballon innerhalb des lebenden Gewebes um oder an den Applikatorschaft legbar ist.

Die intraoperative Bestrahlung (IORT) von lebendem Gewebe ist eine Behandlungsmethode, die besonders nach operativer Entfernung eines Tumors genutzt wird. Der Ballon-Applikator besteht typischerweise aus einem Schaft, wobei am proximalen Ende des Schafts ein Ballon angeordnet ist. Der Schaft dient der Strahlungsquelle als Zugang in das Zentrum des Ballons, um das Tumorbett von innen zu bestrahlen, und zum Einleiten eines Mediums zum Füllen des Ballons. Die Strahlungsquelle ist beispielsweise eine Röntgenstrahlungsquelle (US5621780). Der Applikator wird dabei durch die Haut und das Gewebe hindurch zu dem Tumorbett geführt, wo der Ballon durch Füllung mit einem Medium, typischerweise einem Fluid, besonders einer Kochsalzlösung oder einem Gas, entfaltet wird. Der entfaltete Ballon garantiert die Position und Größe des Tumorbettes relativ zur Strahlungsquelle während der Bestrahlung, da das Tumorbett sonst in sich zusammenfallen würde (WO2005/037363, WO2006/041733, WO2006/065299, WO2006/061722).

Der Ballon kann aus dehnbarem Material bestehen, z.B. Silikon, wodurch die Ballongröße von dem Volumen des Füllmediums abhängt. Nachteilig ist dabei aber die Möglichkeit einer Verformung des Ballons durch mechanische Kräfte von außen.

Der Ballon kann auch aus nichtdehnbarem Material bestehen, z.B. Polyethylenterephthalat (PET) oder Polyurethan (PUR), welches eine diskrete Ballonform und -größe gewährleistet, wodurch die Form der Höhlung des Tumorbetts aufrecht erhalten werden kann. Problematisch beim Einsatz von nichtdehnbaren Materialien ist die Faltung des Ballons vor der Entfernung aus dem Patienten. Es ist möglich, den Ballon mittels spezieller Vorrichtungen aus der entsprechenden Gewebehöhle zu entfernen (US2010/0331878). Alternativ kann der Ballon samt Schaft aus der Gewebehöhle und damit durch das umgebende Gewebe gezogen werden. Beim Absaugen des Füllmediums bildet der Ballon jedoch Falten aus, die abhängig von der Ballongröße mehr oder weniger ausgeprägt sind. Außerhalb des Körpers können solche Falten um den Schaft des Ballon-Applikators gelegt werden, d.h. vor dem Einführen des Ballon-Applikators zum minimal-invasiven Einsatz kann der Ballon des Ballon-Applikators so um den Schaft gelegt werden, dass sich der Ballon-Applikator ohne nennenswerten Widerstand zum Tumorbett führen lässt. Innerhalb des Körpers, also nach Entfaltung des Ballons durch Füllen und Entleeren, ist das wegen der erschwerten Erreichbarkeit nicht möglich. Wird der geleerte, aber nicht um den Schaft gelegte Ballon durch das Gewebe, z.B. durch einen Biopsiekanal, nach außerhalb des Körpers gezogen, drohen Traumatisierungen und Verletzungen des Gewebes.

Weiterhin kann es bei einem Defekt des Schlauchsystems dazu kommen, dass der Ballon nicht entleert werden kann und damit nach erfolgter Bestrahlung in der Gewebehöhlung festsitzt. Das kann dazu führen, dass der Ballon-Applikator chirurgisch entfernt werden muss, was eine unnötige Belastung des Patienten darstellt.

Es besteht damit die Aufgabe, einen Ballon-Applikator bereitzustellen, dessen Ballon innerhalb einer Gewebehöhlung so um oder an den Schaft legbar ist, dass er mit geringem Kraftaufwand durch das Gewebe aus der Gewebehöhlung gezogen werden werden kann.

Diese Aufgabe wird mit einem Ballon-Applikator gemäß Anspruch 1 gelöst. Weitere vorteilhafte Ausführungsformen ergeben sich aus den Nebenansprüchen, den Unteransprüchen, den Figuren und den Ausführungsbeispielen.

Ein erster Aspekt der Erfindung bezieht sich auf einen Ballon-Applikator für eine Bestrahlung eines Hohlraums innerhalb eines lebenden Gewebes, umfassend einen mindestens zweilumigen Schaft mit einem ersten Lumen zum Einleiten eines Mediums zum Füllen eines an einer proximalen und einer distalen Stelle des Schaftes mit diesem verbundenen Ballons und einem zweiten Lumen zum Einführen einer Strahlungsquelle, bei dem der Schaft zwischen der proximalen und der distalen Stelle einen proximal angeordneten ersten Teilbereich und einen distal angeordneten zweiten Teilbereich aufweist, die in einem ersten Arbeitszustand des Ballon-Applikators miteinander verbunden und in einem zweiten Arbeitszustand des Ballon-Applikators voneinander getrennt sind.

Der erfindungsgemäße Ballon-Applikators ist vorteilhaft, weil er im ersten Arbeitszustand die Einführung und Applikation von Strahlung eines Gewebehohlraums ermöglicht und weil im zweiten Arbeitszustand seine Falten seines Ballons innerhalb einer Körperhöhlung durch Steuerung von außen um oder an den Schaft gelegt werden können, wodurch der Ballon-Applikator ohne unnötige Traumatisierungen des Gewebes des Patienten entfernt werden kann. Der erfindungsgemäße Ballon-Applikator ist weiterhin vorteilhaft, weil durch das Drehen der Schaftteile relativ zueinander ggf. das Füllmedium auspressen lässt, wenn der Ballon durch einen Defekt des Schlauchsystems nicht entleert werden kann.

Es ist bevorzugt, wenn der erste Teilbereich des Schafts des Ballon-Applikators die proximale Stelle und der zweite Teilbereich des Schafts die distale Stelle umfasst, an der der Ballon jeweils am Schaft befestigt ist. Vorteilhafterweise kann dabei auf Grund der Verbindung des Ballons mit dem Schaft an der distalen und der proximalen Stelle bei einer Bewegung des ersten Teilbereichs des Schafts um seine Achse relativ zum zweiten Teilbereich des Schafts der Ballon um oder an den Schaft gelegt werden.

Weiterhin weist der Schaft des Ballonapplikators in einem Grenzbereich zwischen dem ersten und dem zweiten Teilbereich des Schafts eine Sollbruchstelle auf, die ausgebildet ist, bei Überschreiten einer definierten Längskraft und/oder eines Torsionsmoments zu brechen. Vorzugsweise befindet sich die Sollbruchstelle an der Grenze zwischen dem ersten und dem zweiten Teil des Schafts. Die Grenze zwischen dem ersten und dem zweiten Teil des Schafts ist dabei als ein Bereich des Schafts zu sehen, der von den Enden des ersten und zweiten Teils jeweils gleich beabstandet ist, also etwa in der Mitte zwischen diesen Enden liegt. An der Sollbruchstelle weist das Material des Schafts Eigenschaften auf, die bei Aufbringen einer Kraft einen leichteren Bruch des Materials als in anderen Bereichen des Schafts ermöglichen. Ein Schaft mit Sollbruchstelle ist vorteilhaft, weil dadurch der proximale und distale Teil relativ zueinander beweglich werden und der Ballon im leeren Zustand innerhalb eines Körpers um den Schaft oder an den Schaft gelegt werden kann. Ein weiter Vorteil besteht darin, dass der Bruch des starren Schaftteils an der Sollbruchstelle eine Mehrfachverwendung des als Einwegprodukt vorgesehenen Ballon- Applikators verhindert, da sich dieser dann nicht mehr in den Körper einführen lässt.

Weiterhin weist der Schaft des Ballon-Applikators in einem Grenzbereich zwischen dem ersten und dem zweiten Teilbereich eine Verbindung mit Gewindegang auf. Ein Gewindegang, z.B. eine Schraubverbindung, ermöglicht vorteilhaft eine Rotationsbewegung der Teilbereiche zueinander und auch eine gleichzeitige Bewegung der Teilbereiche zueinander in Längs- und Rotationsrichtung. Durch die Kombination von Dreh- und Linearbewegung kann der Ballon sowohl um- als auch an den Schaft gelegt werden.

Weiterhin ist es bevorzugt, wenn der Schaft des Ballon-Applikators im Grenzbereich zwischen dem ersten und zweiten Teilbereich eine Abdichtung aufweist, die ausgebildet ist, im zweiten Arbeitszustand des Ballon-Applikators das erste und zweite Lumen des Schafts abzudichten.

In einer bevorzugten Ausführungsform ist in das zweite Lumen des Ballonkatheters zusätzlich ein Werkzeug einführbar, welches ausgebildet ist, mindestens eine Kraft auf den ersten Teilbereichs des Schafts und/oder zweiten Teilbereich des Schafts aufzubringen. Mittels des Werkzeugs lassen sich die Teilbereiche des Schafts bequem voneinander trennen und relativ zueinander bewegen, wodurch der Ballon vorteilhafterweise um den Schaft legbar ist. Der Ballon kann auch manuell, d.h. ohne Werkzeug um den Schaft gelegt werden, beispielsweise indem das aus dem Patienten herausragende Ende gedreht wird, nachdem der erste und zweite Teilbereich des Schafts voneinander getrennt wurden.

Bevorzugt ist der zweite oder erste Teilbereich des Schafts durch das Werkzeug fixierbar und auf den ersten bzw. zweiten Teilbereich des Schafts durch das Werkzeug ein relativ zum zweiten bzw. ersten Teilbereich des Schafts in einer Rotationsrichtung um die Längsachse des ersten Teilbereichs wirkendes Torsionsmoment aufbringbar. Bevorzugt ist auch, wenn der erste oder zweite Teilbereich des Schafts durch das Werkzeug fixierbar und auf den zweiten bzw. ersten Teilbereich des Schafts durch das Werkzeug eine relativ zum ersten bzw. zweiten Teilbereich des Schafts in Längsrichtung des Schafts wirkende Kraft aufbringbar ist. Es können auch beide Teilbereich mittels des Werkzeugs in Rotationsrichtung um ihre jeweilige Längsachse beweglich sein, und zwar in gegenläufiger Richtung zueinander. Weiterhin können auch beide Teilbereiche mittels des Werkzeugs in zueinander entgegengesetzte Längsrichtungen bewegt werden.

Es ist auch eine Kombination von Rotation um die Längsachse des Schafts und Linearbewegung, also Bewegung in Längsrichtung des Schafts, der Teile möglich. Die Kombination von Dreh- und Linearbewegung ist vorteilhaft, weil dadurch der Ballon sowohl um als auch an den Schaft gelegt wird und die Falten des Ballons somit noch effektiver glatt gezogen werden.

Vorzugsweise weist das zweite Lumen des Schafts mindestens eine Vertiefung oder Auswölbung auf, in die das Werkzeug mit mindestens einer Auswölbung bzw. Vertiefung formschlüssig passt. Dadurch wird vorteilhaft bewirkt, dass das Werkzeug nach einem Schlüssel-Schloss-Prinzip einen Formschluss mit dem Schaft eingehen kann. Ist das Werkzeug mehrteilig, passt ein Teil des Werkzeugs formschlüssig mit einer Vertiefung oder Auswölbung zu einer Auswölbung bzw. Vertiefung des ersten Teils des Schafts, und ein anderer Teil des Werkzeugs passt entsprechend formschlüssig zum zweiten Teil des Schafts. So kann ein Teil des Schafts fixiert werden, während der andere bewegt wird. Dadurch wird zum Beispiel eine Rotationsbewegung des einen Teils relativ zum anderen Teil des Schafts ermöglicht.

Das zweite Lumen des Schafts des Ballon-Applikators weist vorzugsweise mindestens einen Haltepunkt zur Aufnahme des Werkzeugs auf. Ein erster Haltepunkt befindet sich vorzugsweise am proximalen Ende des Schafts im zweiten Lumen, d.h. im ersten Teil des Schafts. Der erste Haltepunkt ist vorteilhafterweise so ausgebildet, dass ein Teil des Werkzeugs gegen ihn gedrückt werden kann, d.h. durch das Werkzeug eine in proximaler Längsrichtung des Schafts wirkende Kraft aufbringbar ist.

Ein zweiter Haltepunkt befindet sich vorzugsweise im zweiten Teil des Schafts im zweiten Lumen, wobei er so ausgebildet ist, dass das Werkzeug dort ansetzen kann, um eine Zugkraft in distaler Richtung, d.h. eine in distaler Längsrichtung des Schafts wirkende Kraft, auszuüben. Vorzugsweise weisen der erste und der zweite Haltepunkt Auswölbungen oder Vertiefungen auf, in die das Werkzeug mit entsprechenden Vertiefungen bzw. Auswölbungen formschlüssig passt.

Über die ersten und zweiten Haltepunkte kann das Werkzeug Relativbewegungen des ersten Teils des Schafts gegen den zweiten Teil des Schafts in Längsrichtung bewirkten, wodurch der Abstand zwischen proximaler und distaler Verbindung des Ballons mit dem Schaft vergrößert werden kann. Der Ballon kann damit in Längsrichtung des Schafts glattgezogen und damit an den Schaft angelegt werden.

Vorzugsweise ist ein Teil des Werkzeugs am ersten Haltepunkt fixierbar. Durch einen anderen Teil des Werkzeugs, der am zweiten Haltepunkt im zweiten Lumen des Schafts verbindbar ist, ist eine in distaler Längsrichtung des Schafts wirkende Kraft aufbringbar. Damit wird am Ballon ein Zug in distaler Richtung bewirkt, wodurch eine Relativbewegung des zweiten Teils des Schafts gegen den ersten Teil des Schafts in Längsrichtung bewirkbar ist, wodurch der Abstand zwischen proximaler und distaler Verbindung des Ballons vergrößerbar ist und der Ballon damit entlang des Schafts glattgezogen werden kann.

Es ist ebenfalls bevorzugt, dass ein Teil des Werkzeugs am zweiten Haltepunkt am distalen Ende fixierbar ist und ein anderer Teil des Werkzeugs mit dem ersten Haltepunkt verbunden werden kann, um eine in proximaler Richtung wirkende Kraft aufzubringen und damit den Abstand zwischen proximaler und distaler Verbindung des Ballons zu vergrößern und den Ballon glattzuziehen. Es ist weiterhin möglich, dass gleichzeitig am zweiten Haltepunkt eine Zugkraft und am ersten Haltepunkt eine Druckkraft aufbringbar ist, um den Abstand zwischen proximaler und distaler Verbindung des Ballons zu vergrößern und den Ballon glattzuziehen.

Weiterhin weist der Ballon-Applikator sowohl Vertiefungen und/oder Auswölbungen auf, in die das Werkzeug formschlüssig passt, um eine Rotationsbewegung des ersten oder zweiten Teils des Schafts relativ zum zweiten bzw. ersten zu bewirken, als auch Haltepunkte für das Werkzeug, um eine Längsbewegung des ersten oder zweiten Teils des Schafts relativ zum zweiten bzw. ersten zu bewirken. Diese Ausführungsform ist vorteilhaft, weil sie relative Rotations- und Längsbewegungen der Schaftteile ermöglichen, die sowohl gleichzeitig als auch nacheinander ausführbar sind. Mit anderen Worten ist es bevorzugt, wenn das Werkzeug und das Lumen des Schafts zueinander passende Vertiefungen und Auswölbungen aufweisen, die sowohl eine Rotationsbewegung um die als auch Längsbewegungen entlang der Längsachse des Schafts ermöglichen.

Weiterhin ist es bevorzugt, wenn der erfindungsgemäße Ballon-Applikator einen starren und einen flexiblen Abschnitt aufweist. Dabei ist der starre Abschnitt der, der in die Gewebehöhle eingebracht wird und am dem der Ballon befestigt ist. Der erste und zweite Teil des Ballons mit den Ballonbefestigungen werden dementsprechend von dem starren Abschnitt umfasst. Der flexible Teil vorgesehen, sich teilweise z.B. in einem Biopsiekanal, durch den der Ballon-Applikator in die Gewebehöhle gebracht wurde, und teilweise außerhalb des Körpers zu befinden; er dient zur Handhabung des Ballon-Applikators.

Ein Werkzeug zum Aufbringen einer Kraft auf den ersten Teilbereich des Schafts und/oder den zweiten Teilbereich des Schafts des erfindungsgemäßen Ballon-Applikators weist eine äußere Röhre und einen in der äußeren Röhre verschiebbar gelagerten inneren Kern auf. Das Werkzeug ist nach dem Entfernen der Strahlungsquelle aus dem zweiten Lumen in das zweite Lumen einführbar und in diesem in Längsrichtung verschiebbar beweglich.

Die äußere Röhre und der innere Kern des Werkzeugs können aus jedem möglichen Material bestehen, das die Verwendung in einer Gewebehöhlung erlaubt. Weiterhin können die äußere Röhre und der innere Kern des Werkzeugs aus demselben Material bestehen, können aber auch unterschiedliche Materialien aufweisen.

Die äußere Röhre und der innere Kern des Werkzeugs sind vorteilhafterweise stabartig ausgebildet. Die stabartige Ausbildung ermöglicht die Einführung in das zweite Lumen des Ballon-Applikators und auch die Fixierung und Bewegung des ersten und zweiten Teilbereichs des Schafts durch das Werkzeug.

Die äußere Röhre des Werkzeugs weist dabei mindestens eine Auswölbung oder Vertiefung auf, die formschlüssig zu mindestens einer Vertiefung bzw. Auswölbung im zweiten Lumen des Ballon-Applikators passen. Dadurch wird vorteilhaft bewirkt, dass im Schaftinneren die äußere Röhre nach einem Schlüssel-Schloss-Prinzip einen Formschluss mit dem zweiten Teilbereich des Schafts eingehen kann.

Der innere Kern des Werkzeugs weist mindestens eine Auswölbung oder Vertiefung auf, die formschlüssig zu mindestens einer Vertiefung bzw. Auswölbung im zweiten Lumen des Ballon-Applikators passen. Dadurch wird vorteilhaft bewirkt, dass im Schaftinneren der innere Kern nach einem Schlüssel-Schloss-Prinzip einen Formschluss mit dem ersten Teilbereich des Schafts eingehen kann.

Ein zweiter Aspekt der Erfindung betrifft ein System aus dem erfindungsgemäßen Ballon-Applikator und dem Werkzeug. In dem System kann das Werkzeug vorteilhaft in dem zweiten Lumen des Schafts des Ballon-Applikators bewegt werden, nachdem die Strahlungsquelle entfernt worden ist. Durch das Werkzeug sind vorteilhaft Trennung des ersten und zweiten Teilbereich des Schafts voneinander sowie Bewegungen des ersten und des zweiten Teils des Schafts relativ zueinander bewirkbar.

Bei einem Verfahren zum Glätten von Falten eines am Schaft eines Ballon-Applikators angeordneten Ballons innerhalb eines lebenden Gewebes wird ein erster Teil des Schafts relativ zu einem zweiten Teil des Schafts bewegt und der Ballon um oder an den Schaft des Ballon-Applikators gelegt. Nach Entfernung einer Strahlungsquelle aus einem zweiten Lumen des Ballon-Applikators wird ein Werkzeug in das zweite Lumen des Ballon-Applikators eingeführt. Ein erster Teilbereich des Schafts des Ballon-Applikators wird durch das Werkzeug fixiert und auf einen zweiten Teilbereich eine Kraft aufgebracht. Dabei kommt es zum Bruch einer im Schaft des Ballon-Applikators vorgesehenen Sollbruchstelle, wodurch eine Relativbewegung des zweiten Teils zum ersten Teil des Werkzeugs ermöglicht wird. Der zweite Teilbereich wird dann relativ zum ersten Teilbereich mittels des Werkzeugs bewegt. Die Bewegung kann in Rotationsrichtung um die und/oder entlang der Längsachse des Schafts des Ballon-Applikators erfolgen. Alternativ können die Ballonfalten auch ohne Werkzeug um oder an den Schaft gelegt werden, indem von außen manuell an dem aus dem Körper herausragenden Abschnitt gedreht wird. Dabei kommt es ebenfalls zum Bruch der Sollbruchstelle.

Durch die Relativbewegung der Teilbereiche durch Rotation und/oder Längsbewegung der Teilbereiche des Schafts wird der Ballon um den Schaft herumgelegt und/oder entlang des Schafts an den Schaft angelegt. Besteht die Verbindung zwischen dem ersten und dem zweiten Teil des Schafts in einem Gewindegang, wird gleichzeitig eine Rotationsbewegung um die und eine Längsbewegung entlang der Längsachse des Schafts bewirkt, wodurch der Ballon gleichzeitig um den Schaft gewickelt wird und am Schaft entlang an den Schaft angelegt wird.

Die Erfindung wird anhand der Figuren näher erläutert. Es zeigen
- **Figur 1**: eine beispielhafte Ausführungsform eines erfindungsgemäßen Ballon-Applikators in Querschnittsansicht.
- **Figur 2**: eine beispielhafte Ausführungsform eines erfindungsgemäßen Werkzeugs in Querschnittsansicht.
- **Figur 3**: eine beispielhafte Ausführungsform eines erfindungsgemäßen Systems in Querschnittsansicht.
- **Figur 4**: eine herkömmlichen Ballon-Applikator in Querschnittsansicht.
- **Figur 5**: einen herkömmlichen Ballon-Applikator, von der proximalen Seite her gesehen.

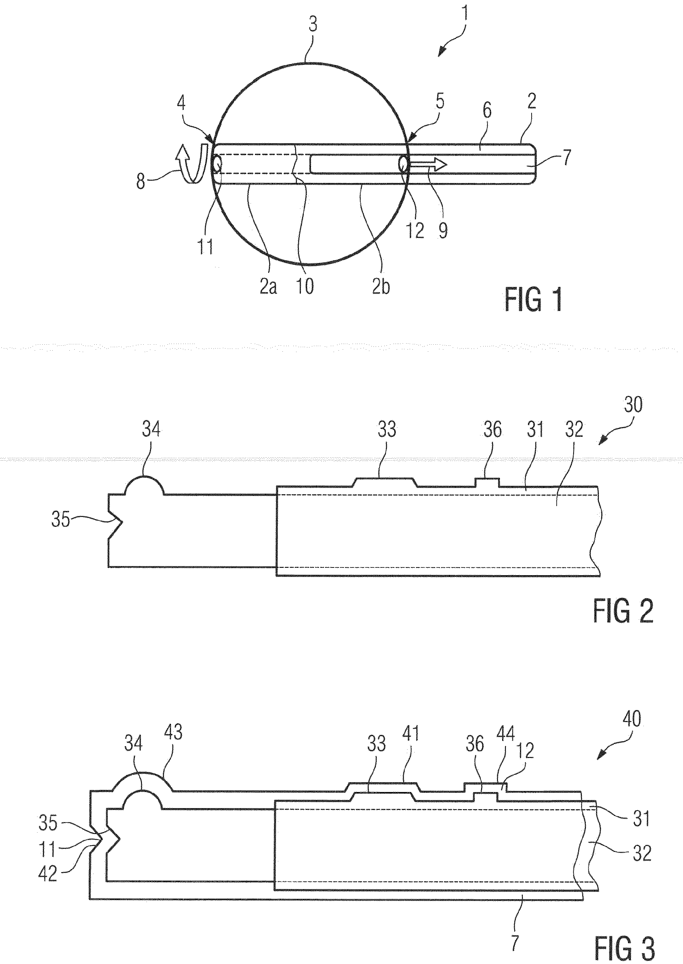

Die in Figur 1 gezeigten beispielhaften Ausführungsform des erfindungsgemäßen Ballon-Applikators 1 für eine Bestrahlung eines Hohlraums innerhalb eines lebenden Gewebes umfasst einen mindestens zweilumigen Schaft 2, einen an einer proximalen 4 und einer distalen Stelle 5 des Schaftes 2 mit diesem verbundenen Ballon 3, einem ersten Lumen 6 zum Einleiten und Ausleiten eines Mediums zum Füllen des Ballons 3 und einem zweiten Lumen 7 zum Einführen einer Strahlungsquelle, wobei durch das zweite Lumen 7 nach Entfernen der Strahlungsquelle zusätzlich ein Werkzeug 30 einführbar ist, welches ausgebildet ist, eine Bewegung eines proximalen angeordneten ersten Teilbereichs des Schafts 2a relativ zu einem distal angeordnetem zweiten Teilbereichs des Schafts 2b zu bewirken. Der Schaft 2 weist eine Sollbruchstelle 10 auf, an der das Material des Schafts 2 zwischen erstem Teilbereich 2a und zweitem Teilbereich 2b gebrochen und damit die Verbindung zwischen den beiden Teilen des Schafts 2 gelöst werden kann. In einer alternativen Ausführungsform kann an Stelle der Sollbruchstelle 10 eine Verbindung durch einen Gewindegang, d.h. z.B. durch eine Schraubverbindung, vorhanden sein. Weiterhin ist auch eine magnetische Verbindung zwischen dem ersten Teilbereich 2a und zweiten Teilbereich 2b möglich. Im zweiten Lumen 7 ist proximal ein erster Haltepunkt 11 zur Aufnahme des Werkzeugs 30 angeordnet, und distal ein zweiter Haltepunkt 12, der ebenfalls zur Aufnahme des Werkzeugs 30 dient.

Das in Figur 2 dargestellte Werkzeug 30 besteht im Wesentlichen aus einer äußeren Röhre 31 und einem inneren Kern 32. Der innere Kern 32 ist in der äußeren Röhre 31 verschiebbar gelagert. In einer stark vereinfachten Form kann das Werkzeug 30 auch ein Stab sein, der innerhalb des zweiten Lumens 7 in Längsrichtung bewegt werden kann. Die äußere Röhre 31 weist Auswölbungen 33 und 36 auf, und der innere Kern 32 eine Auswölbung 34 und eine Vertiefung 35.

In Figur 3 ist ein durch den Ballon-Applikator 1 und das Werkzeug 30 gebildetes System 40 dargestellt. Die äußere Röhre 31 weist eine Auswölbung 33 auf, die formschlüssig in eine Vertiefung 41 im Material des zweiten Lumens 7 passt. Der innere Kern 32 weist eine Auswölbung 34 auf, die formschlüssig in eine Vertiefung 43 im Material des zweiten Lumens 7 passt. Die Auswölbungen im Werkzeug 30 und Vertiefungen im zweiten Lumen 7 entsprechen dabei idealerweise in der Zahl einander. Im Werkzeug 30 können auch Vertiefungen und im zweiten Lumen 7 Auswölbungen vorhanden sein. Es können auch sowohl Auswölbungen als auch Vertiefungen im Werkzeug 30 und im zweiten Lumen 7 vorhanden sein. Dabei weisen Werkzeug 30 und Schaft 2 mindestens eine Auswölbung bzw.

Vertiefung auf, die jede passende Form haben kann, also z.B. rund oder kantig, quadratisch oder länglich ausgebildet sein kann, wobei eine längliche, nutenartige Ausbildung bevorzugt ist. Die Zahl der Auswölbungen bzw. Vertiefungen kann zweckgemäß beliebig hoch sein, vorzugsweise 1 - 10, ebenfalls bevorzugt 2 - 9, ebenfalls bevorzugt 3 - 8, ebenfalls bevorzugt 4 - 7, und ebenfalls bevorzugt 5 oder 6.

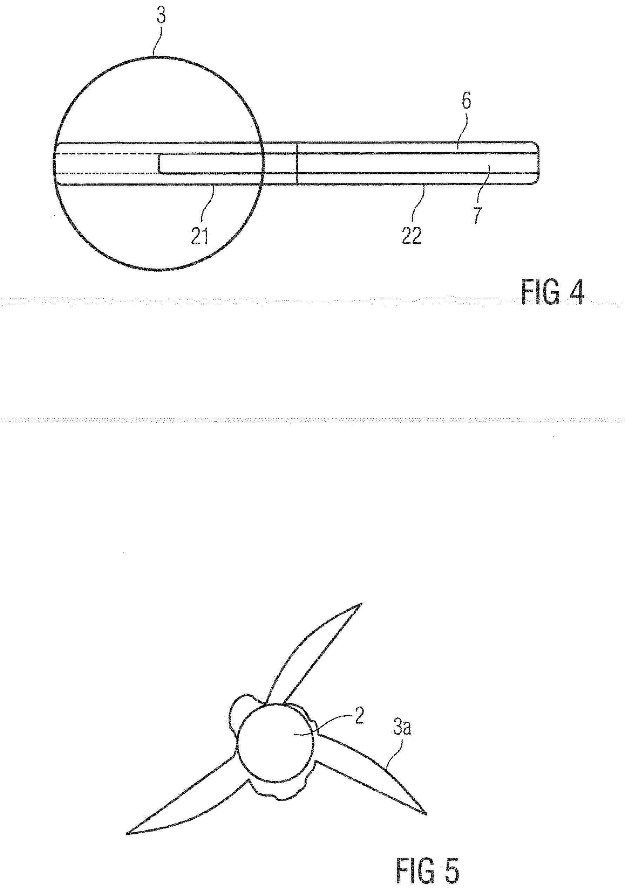

Der Ballon-Applikator weist über den starren Abschnitt 21 hinaus einen flexiblen Abschnitt 22 auf, wie in Figur 4 dargestellt. Der erste Teil 2a und der zweite Teil 2b des Schafts 2 sind vom starren Abschnitt 21 umfasst. Der flexible Abschnitt 22 ist idealerweise länger als der starre Abschnitt 21, da er vorgesehen ist, sich während einer Behandlung z.B. in einem Biopsiekanal und außerhalb des Patientenkörpers zu befinden, während sich der starre Abschnitt 21 in einer Gewebehöhlung befinden soll.

Wie in den Figuren 2 und 3 dargestellt, weist der innere Kern 32 weiterhin mindestens eine Vertiefung 35 am Haltepunkt 11 auf, die formschlüssig zu einer Auswölbung 42 im zweiten Lumen 7 passt. Die Form der Vertiefung 35 ist dabei eckig, so dass rutschfeste Kanten vorhanden sind. Der innere Kern 32 des Werkzeugs 30 kann somit am Haltepunkt 11 ansetzen und eine Rotationsbewegung um seine Längsachse, und damit auch eine Rotationsbewegung des Schafts 2 um seine Längsachse, bewirken, ohne vom Haltepunkt abzurutschen. Das zweite Lumen 7 weist dabei am Haltepunkt mindestens eine Vertiefung und der innere Kern 32 mindestens eine Auswölbung auf, die formschlüssig zueinander passen. Die Zahl der Auswölbungen bzw. Vertiefungen kann zweckgemäß beliebig hoch sein, vorzugsweise 1 - 10, ebenfalls bevorzugt 2 - 9, ebenfalls bevorzugt 3 - 8, ebenfalls bevorzugt 4 - 7, und ebenfalls bevorzugt 5 oder 6.

Ist der Ballon 3 vom Medium entleert, liegt er in Falten 3a vor, wie in Figur 5 dargestellt. Die Falten des entleerten Ballons 3a müssen an den Schaft 2 gelegt oder glatt gezogen werden, damit der Ballon 3 aus der Gewebehöhlung entfernt werden kann.

Um den Ballon 3 des Ballon-Applikators 1 um den Schaft 2 zu legen, kann der zweite Teil 2b des Schafts 2 mit der äußeren Röhre 31 des Werkzeugs 30 fixiert und der erste Teil 2a des Schafts 2 mit dem inneren Kern 32 relativ zum zweite Teil 2b in Richtung der Rotationsbewegung 8 ein Torsionsmoment aufgebracht werden. Dazu wird nach Entfernung einer Strahlungsquelle aus dem zweiten Lumen 7 das Werkzeug 30 in das zweite Lumen 7 eingeführt, die äußere Röhre 31 mittels der Auswölbung 33 in der Vertiefung 41 im zweiten Lumen 7 mit dem zweiten Teil des Schafts 2b formschlüssig verbunden, und der innere Kern 32 mittels der Auswölbung 34 in der Vertiefung 43 mit dem proximalen Teil 2a des Schafts formschlüssig verbunden. Durch eine Rotationsbewegung 8 des inneren Kerns 32 und des dadurch auf den ersten Teilbereich des Schafts 2a wirkenden Torsionsmoments kommt es zum Bruch der Sollbruchstelle 10, wodurch eine Relativbewegung des ersten Teilbereichs 2a gegenüber dem zweiten Teilbereich des Schafts 2b ermöglicht wird. Durch eine weitere, mittels des inneren Kerns 32 bewirkte Rotationsbewegung 8 des ersten Teilbereichs 2a wird der Ballon 3 um den Schaft 2 gelegt.

Soll der Ballon durch Glattziehen an den Schaft gelegt werden, kann dies durch eine lineare, mit dem Werkzeug 30 bewirkte Bewegung realisiert werden. Dazu wird der innere Kern 32 mit der Vertiefung 35 am Haltepunkt 11 mit der Auswölbung 42 im zweiten Lumen 7 formschlüssig verbunden und als Gegenlager genutzt, die äußere Röhre 31 über die Auswölbung 36 mit der Vertiefung 44 im Lumen 7 am Haltepunkt 12 formschlüssig verbunden und auf die äußere Röhre in distaler Richtung eine Zugkraft ausgeübt. Durch die wirkende Kraft kommt es zum Bruch des Materials des Schafts 2 der Sollbruchstelle 10. Diese lineare Bewegung 9 ist in Figur 1 beispielhaft dargestellt. Durch weiteren Zug in Richtung des Haltepunkts 12 werden die Teilbereiche 2a und 2b auseinander und der Ballon glatt gezogen.

Alternativ kann der Ballon auch in proximaler Richtung gestreckt werden. Dazu wird der innere Kern 32 über seine Vertiefung 35 mit der Auswölbung 42 am ersten Haltepunkt 11 formschlüssig verbunden und in Richtung des Haltepunkts 11 gedrückt. Am Haltepunkt 12 wird die äußere Röhre 31 des Werkzeugs 30 über ihre Auswölbung 36 mit der Vertiefung 44 formschlüssig verbunden, um den distalen Teibereich 2b zu fixieren, damit nicht der gesamte Ballon-Applikator 1 durch die auf den Haltepunkt 11 wirkende Kraft weiter ins Gewebe hineingedrückt wird. Durch die wirkende Kraft kommt es zum Bruch des Materials des Schafts 2 der Sollbruchstelle 10. Durch weiteren Druck in Richtung des Haltepunkts 11 werden die Teile 2a und 2b auseinander und der Ballon 3 glatt gezogen. Es kann auch kombiniert gleichzeitig am Haltepunkt 11 gedrückt und am Haltepunkt 12 gezogen werden.

Es ist auch möglich, zuerst das Material des Schafts 2 zwischen erstem Teilbereich 2a und zweitem Teilbereich 2b durch ein Torsionsmoment 8 des inneren Kerns 32 zu brechen und dann durch Druck und/oder Zug mittels des inneren Kerns 32 in proximaler oder distaler Längsrichtung des Schafts 2 den Ballon glatt zu ziehen. Die durch das Torsionsmoment bewirkte Rotationsbewegung 8 kann dabei auch weitergeführt werden.

Besteht die Verbindung zwischen dem ersten Teilbereich 2a und dem zweiten Teilbereich 2b des Schafts 2 in einem Gewindegang, wird gleichzeitig eine Rotationsbewegung 8 um die und eine Längsbewegung 9 entlang der Längsachse des Schafts 2 bewirkt, wodurch der Ballon 3 gleichzeitig um den Schaft 2 gewickelt wird und am Schaft 2 entlang angelegt wird.

Für einen Fachmann naheliegende Abwandlungen und Änderungen der Erfindung fallen unter den Schutzumfang der Patentansprüche.

### Bezugszeichenliste

- 1 =: Ballon-Applikator
- 2 =: Schaft
- 2a =: erster Teilbereich des Schafts
- 2b =: zweiter Teilbereich des Schafts
- 3 =: Ballon
- 3a =: entleerter Ballon
- 4 =: proximale Verbindungsstelle von Ballon und Schaft
- 5 =: distale Verbindungsstelle von Ballon und Schaft
- 6 =: erstes Lumen
- 7 =: zweites Lumen
- 8 =: Indikation der Rotationsbewegung
- 9 =: Indikation der Längsbewegung
- 10 =: Sollbruchstelle
- 11 =: erster Haltepunkt
- 12 =: zweiter Haltepunkt
- 21 =: starrer Abschnitt des Schafts
- 22 =: flexibler Abschnitt des Schafts
- 30 =: Werkzeug
- 31 =: äußere Röhre des Werkzeugs
- 32 =: innerer Kern des Werkzeugs
- 33 =: Auswölbung der äußeren Röhre
- 34 =: Auswölbung des inneren Kerns
- 35 =: Vertiefung des inneren Kerns
- 36 =: Auswölbung der äußeren Röhre
- 40 =: System aus Ballon-Applikator und Werkzeug
- 41 =: Vertiefung im zweiten Lumen des Ballon-Applikators
- 42 =: Auswölbung im zweiten Lumen des Ballon-Applikators
- 43 =: Vertiefung im zweiten Lumen des Ballon-Applikators
- 44 =: Vertiefung im zweiten Lumen des Ballon-Applikators

## Patentansprüche

1. Ballon-Applikator (1) für eine Bestrahlung eines Hohlraums innerhalb eines lebenden Gewebes, umfassend einen mindestens zweilumigen Schaft (2) mit einem ersten Lumen (6) zum Einleiten eines Mediums zum Füllen eines an einer proximalen (4) und einer distalen Stelle (5) des Schaftes (2) mit diesem verbundenen Ballons (3) und einem zweiten Lumen (7) zum Einführen einer Strahlungsquelle,
bei dem der Schaft (2) zwischen der proximalen (4) und der distalen Stelle (5) einen proximal angeordneten ersten Teilbereich (2a) und einen distal angeordneten zweiten Teilbereich (2b) aufweist, die in einem ersten Arbeitszustand des Ballon-Applikators (1) miteinander verbunden und in einem zweiten Arbeitszustand des Ballon-Applikators (1) voneinander getrennt sind
**dadurch gekennzeichnet, dass**
der Schaft (2) in einem Grenzbereich zwischen dem ersten (2a) und dem zweiten Teilbereich des Schafts (2b) eine Sollbruchstelle (10) aufweist, die ausgebildet ist, bei Überschreiten einer definierten Längskraft und/oder eines Torsionsmoments zu brechen, oder der Schaft (2) in dem Grenzbereich zwischen dem ersten (2a) und dem zweiten Teilbereich (2b) eine Verbindung mit Gewindegang aufweist.

2. Ballon-Applikator (1) nach Anspruch 1, bei dem der erste Teilbereich des Schafts (2a) die proximale Stelle (4) und der zweite Teilbereich des Schafts (2b) die distale Stelle (5) umfasst, an der der Ballon (3) jeweils am Schaft (2) befestigt ist.

3. Ballon-Applikator (1) nach einem der Ansprüche 1 oder 2, bei dem der Schaft (2) im Grenzbereich zwischen dem ersten (2a) und zweiten Teilbereich (2b) eine Abdichtung aufweist, die ausgebildet ist, im zweiten Arbeitszustand des Ballon-Applikators (1) das erste (6) und zweite Lumen des Schafts (7) abzudichten.

4. Ballon-Applikator (1) nach einem der vorherigen Ansprüche, bei dem das zweite Lumen (7) eingerichtet ist, zusätzlich ein Werkzeug (30) einzuführen, welches ausgebildet ist, mindestens eine Kraft auf den ersten Teilbereichs des Schafts (2a) und/oder zweiten Teilbereich des Schafts (2b) aufzubringen.

5. Ballon-Applikator (1) nach Anspruch 4, bei dem der zweite (2b) oder erste Teilbereich des Schafts (2a) durch das Werkzeug (30) fixierbar ist und auf den ersten (2a) bzw. zweiten Teilbereich des Schafts (2b) durch das Werkzeug (30) ein relativ zum zweiten (2b) bzw. ersten Teilbereich des Schafts (2a) in einer Rotationsrichtung (8) um die Längsachse des ersten Teilbereichs wirkendes Torsionsmoment aufbringbar ist.

6. Ballon-Applikator (1) nach Anspruch 4 oder 5, bei dem der erste (2a) oder zweite Teilbereich des Schafts (2b) durch das Werkzeug (30) fixierbar ist und auf den zweiten (2b) bzw. ersten Teilbereich des Schafts (2a) durch das Werkzeug (30) eine relativ zum ersten (2a) bzw. zweiten Teilbereich des Schafts (2b) in Längsrichtung des Schafts (2) wirkende Kraft aufbringbar ist.

7. Ballon-Applikator (1) nach einem der Ansprüche 4 - 6, bei dem das zweite Lumen (7) mindestens eine Vertiefung (41, 43, 44) oder Auswölbung aufweist, in die das Werkzeug (30) mit mindestens einer Auswölbung (33, 34, 36) oder Vertiefung formschlüssig passt, und / oder bei dem das zweite Lumen (7) mindestens einen Haltepunkt (11, 12) zur Aufnahme des Werkzeugs (30) aufweist.

8. Ballon-Applikator (1) nach einem der vorherigen Ansprüche, bei dem der Schaft (2) einen starren (21) und einen flexiblen Abschnitt (22) aufweist.

9. System (40) aus Ballon-Applikator (1) gemäß einem der Ansprüche 1 - 8 und einem Werkzeug (30) zum Aufbringen einer Kraft auf den ersten Teil des Schafts (2a) und/oder zweiten Teil des Schafts (2b) des Ballon-Applikators (1), das eine äußere Röhre (31) und einen in der äußeren Röhre (31) verschiebbar gelagerten inneren Kern (32) aufweist.

## Claims

1. Balloon applicator (1) for irradiation of a cavity within living tissue, comprising an at least double-lumen shaft (2) with a first lumen (6) for introduction of a medium for filling a balloon (3) connected to the shaft (2) at a proximal (4) and a distal location (5) thereof, and with a second lumen (7) for insertion of a radiation source,
in which the shaft (2), between the proximal (4) and the distal location (5), has a proximally arranged first partial region (2a) and a distally arranged second partial region (2b) which, in a first working state of the balloon applicator (1), are connected to each other and, in a second working state of the balloon applicator (1), are separated from each other,
**characterized in that**
the shaft (2), in a boundary region between the first (2a) and the second partial region of the shaft (2b), has a predetermined breaking point (10) which is designed to break when a defined longitudinal force and/or a torsional moment are/is exceeded, or the shaft (2), in the boundary region between the first (2a) and the second partial region (2b), has a connection with a thread turn.

2. Balloon applicator (1) according to Claim 1, in which the first partial region of the shaft (2a) comprises the proximal location (4) and the second partial region of the shaft (2b) comprises the distal location (5), at which the balloon (3) is respectively secured on the shaft (2).

3. Balloon applicator (1) according to either of Claims 1 and 2, in which the shaft (2), in the boundary region between the first (2a) and second partial region (2b), has a seal which is designed to seal the first (6) and second lumen of the shaft (7) in the second working state of the balloon applicator (1).

4. Balloon applicator (1) according to one of the preceding claims, in which the second lumen (7) is configured for additional insertion of a tool (30) which is designed to apply at least one force to the first partial region of the shaft (2a) and/or second partial region of the shaft (2b).

5. Balloon applicator (1) according to Claim 4, in which the second (2b) or first partial region of the shaft (2a) can be fixed by the tool (30), and a torsional moment acting relative to the second (2b) or first partial region of the shaft (2a) in a rotational direction (8) about the longitudinal axis of the first partial region can be applied by the tool (30) to the first (2a) or second partial region of the shaft (2b).

6. Balloon applicator (1) according to Claim 4 or 5, in which the first (2a) or second partial region of the shaft (2b) can be fixed by the tool (30), and a force acting relative to the first (2a) or second partial region of the shaft (2b) in the longitudinal direction of the shaft (2) can be applied by the tool (30) to the second (2b) or first partial region of the shaft (2a).

7. Balloon applicator (1) according to one of Claims 4-6, in which the second lumen (7) has at least one depression (41, 43, 44) or protuberance in which the tool (30) engages with a form fit via at least one protuberance (33, 34, 36) or depression, and/or in which the second lumen (7) has at least one holding point (11, 12) for receiving the tool (30).

8. Balloon applicator (1) according to one of the preceding claims, in which the shaft (2) has a rigid (21) and a flexible portion (22).

9. System (40) composed of balloon applicator (1) according to one of Claims 1-8 and of a tool (30) for applying a force to the first part of the shaft (2a) and/or second part of the shaft (2b) of the balloon applicator (1), which tool (30) has an outer tube (31) and an inner core (32) mounted movably in the outer tube (31).

## Revendications

1. Applicateur de ballonnet (1) pour une exposition à un rayonnement d'une cavité à l'intérieur d'un tissu vivant, comprenant une tige (2) à au moins deux lumières, munie d'une première lumière (6) pour l'introduction d'un milieu pour le remplissage d'un ballonnet (3) relié à la tige (2) en un point proximal (4) et en un point distal (5) de celle-ci, et d'une deuxième lumière (7) pour l'introduction d'une source de rayonnement,
dans lequel la tige (2) présente, entre le point proximal (4) et le point distal (5), une première zone partielle agencée de manière proximale (2a) et une deuxième zone partielle agencée de manière distale (2b), qui sont reliées l'une à l'autre dans un premier état de travail de l'applicateur de ballonnet (1) et séparées l'une de l'autre dans un deuxième état de travail de l'applicateur de ballonnet (1),
**caractérisé en ce que**
la tige (2) présente, dans une zone limite entre la première (2a) et la deuxième zone partielle de la tige (2b), un point destiné à la rupture (10), qui est configuré pour se rompre en cas de dépassement d'une force longitudinale définie et/ou d'un couple de torsion, ou la tige (2) présente, dans la zone limite entre la première (2a) et la deuxième zone partielle (2b), une liaison avec un pas de filetage.

2. Applicateur de ballonnet (1) selon la revendication 1, dans lequel la première zone partielle de la tige (2a) comprend le point proximal (4) et la deuxième zone partielle de la tige (2b) comprend le point distal (5), où le ballonnet (3) est respectivement fixé à la tige (2).

3. Applicateur de ballonnet (1) selon l'une quelconque des revendications 1 ou 2, dans lequel la tige (2) présente, dans la zone limite entre la première (2a) et la deuxième zone partielle (2b), un joint d'étanchéité, qui est configuré pour étanchéifier la première (6) et la deuxième lumière de la tige (7) dans le deuxième état de travail de l'applicateur de ballonnet (1).

4. Applicateur de ballonnet (1) selon l'une quelconque des revendications précédentes, dans lequel la deuxième lumière (7) est conçue pour introduire en outre un outil (30), qui est configuré pour appliquer au moins une force sur la première zone partielle de la tige (2a) et/ou la deuxième zone partielle de la tige (2b).

5. Applicateur de ballonnet (1) selon la revendication 4, dans lequel la deuxième (2b) ou la première zone partielle de la tige (2a) peut être fixée par l'outil (30) et un couple de torsion agissant par rapport à la deuxième (2b) ou à la première zone partielle de la tige (2a) dans une direction de rotation (8) autour de l'axe longitudinal de la première zone partielle peut être appliqué par l'outil (30) sur la première (2a) ou la deuxième zone partielle de la tige (2b).

6. Applicateur de ballonnet (1) selon la revendication 4 ou 5, dans lequel la première (2a) ou la deuxième zone partielle de la tige (2b) peut être fixée par l'outil (30) et une force agissant par rapport à la première (2a) ou à la deuxième zone partielle de la tige (2b) dans la direction longitudinale de la tige (2) peut être appliquée par l'outil (30) sur la deuxième (2b) ou la première zone partielle de la tige (2a).

7. Applicateur de ballonnet (1) selon l'une quelconque des revendications 4 à 6, dans lequel la deuxième lumière (7) présente au moins un creux (41, 43, 44) ou un bombement, dans lequel l'outil (30) s'adapte par complémentarité de forme avec au moins un bombement (33, 34, 36) ou un creux, et/ou dans lequel la deuxième lumière (7) présente au moins un point de retenue (11, 12) pour la réception de l'outil (30).

8. Applicateur de ballonnet (1) selon l'une quelconque des revendications précédentes, dans lequel la tige (2) présente une section rigide (21) et une section flexible (22).

9. Système (40) composé d'un applicateur de ballonnet (1) selon l'une quelconque des revendications 1 à 8 et d'un outil (30) pour l'application d'une force sur la première partie de la tige (2a) et/ou la deuxième partie de la tige (2b) de l'applicateur de ballonnet (1), qui présente un tube extérieur (31) et un noyau intérieur (32) logé de manière coulissante dans le tube extérieur (31).
